# EUROPEAN PATENT SPECIFICATION

(11) **EP 2 864 334 B1**
(45) Date of publication and mention of the grant of the patent: **04.01.2017**
(21) Application number: 12772546.3
(22) Date of filing: 25.06.2012
(51) Int. Cl.: C07D 491/052, A61K 31/407, A61P 31/14

(54) **THIOSEMICARBAZIDES AND TRIAZOLES DERIVED FROM ETODOLAC AND THEIR SYNTHESES**
AUS ETODOLAC ABGELEITETEN THIOSEMICARBAZIDEN UND TRIAZOLEN UND DEREN HERSTELLUNG
THIOSEMICARBAZIDES ET DE TRIAZOLES DÉRIVÉS DE L'ÉTODOLAC ET LEURS SYNTHÈSES

(43) Date of publication of application: 29.04.2015
(73) Proprietor: Kucukguzel, S. Guniz, 34718 Istanbul (TR)
(72) Inventor: SUZGUN, Pelin, 34381 Istanbul (TR)
(74) Representative: Mutlu, Aydin
(86) International application number: PCT/TR2012/000109
(87) International publication number: WO 2014/003694

(56) References cited:
- WO-A1-03/099275
- GOPLSAMY A ET AL: "Discovery of Pyrano[3,4-b]indoles as Potent and Selective HCV NS5B Polymerase Inhibitors", JOURNAL OF MEDICINAL CHEMISTRY, AMERICAN CHEMICAL SOCIETY, US, vol. 47, 26 December 2004 (2004-12-26), pages 6603-6608, XP002361050, ISSN: 0022-2623, DOI: 10.1021/JM0401255
- KUS C ET AL: "Synthesis and antioxidant properties of novel N-methyl-1,3,4-thiadiazol-2-amine and 4-methyl-2H-1,2,4-triazole-3(4H)-thione derivatives of benzimidazole class", BIOORGANIC & MEDICINAL CHEMISTRY, PERGAMON, GB, vol. 16, no. 8, 15 April 2008 (2008-04-15) , pages 4294-4303, XP022617879, ISSN: 0968-0896, DOI: 10.1016/J.BMC.2008.02.077 [retrieved on 2008-02-29]
- ÖZADALI ET AL: "Synthesis and biological evaluation of isoxazolo[4,5-]pyridazin-4-(5)-one analogues as potent anti-inflammatory agents", BIOORGANIC & MEDICINAL CHEMISTRY, PERGAMON, GB, vol. 20, no. 9, 7 March 2012 (2012-03-07), pages 2912-2922, XP028412819, ISSN: 0968-0896, DOI: 10.1016/J.BMC.2012.03.021 [retrieved on 2012-03-15]

## Description

### FIELD OF THE INVENTION

Etodolac (*R,S*)-2-[1,8-diethyl-1,3,4-tetrahydrapyrano[3,4-*b*]indol-1-yl]acetic acid is a non-steroidal antiinflammatory drug which has antitumor activities reported in the literature. In light of this, etodolac was chosen as as a starting compound to synthesize thiosemicarbazides and 1,2,4-triazoles, as etodolac contains carboxylic acid group with the structure of pyrano[3,4-*b*]indole. Characterization of the compounds synthesized from etodolac, were performed by the help of elemental analysis, UV, FT-IR, ¹H-NMR, ¹³C-NMR and HR-MS spectral data while the purities of them were proved with TLC.

### BACKGROUND OF THE INVENTION

Etodolac (*R,S*)-2-[1,8-diethyl-1,3,4-tetrahydrapyrano[3,4-*b*]indol-1-yl)acetic acid is a nonsteroidal antiinflammatory agent with analgesic and antipyretic properties. Etodolac has a stronger inhibitory effect on cyclooxygenase-2 (COX-2) activation. Its mechanism of action is through inhibition of COX with reduction in the synthesis of prostaglandins and other mediators of inflammation and pain. Prostaglandins are thought to play an important role in the proliferation of prostate cancer and are highly expressed in prostate cancer tissue. COX-2 inhibitors such as etodolac, suppress proliferation, induce apoptosis in prostate cancer cells and have no effect in normal prostate stromal cells.

Thiosemicarbazides are interesting compounds with essential structural features responsible for various biological activities. Basic cyclisation of thiosemicarbazides yields in 1,2,4-triazoles, which also display a wide range of interesting biological activities. For instance, the search for more effective and less toxic antitumoral drugs, led to the discovery of 1,2,4-triazoles derivatives having antitumoral activity.

Compounds, containing the pyrano[3,4-*b*]indole scaffold have been reported to exhibit anti-hepatitis C virus (HCV) NS5B polymerase activity. HCV NS5B RNA polymerase has crucial role for replicating the viral RNA genome and is a key target for the development of anti-HCV drugs. Gopalsamy et al. recently identified a novel series of 1,3,4,9-tetrahydrapyrano[3,4-*b*]indole derivatives as potent and selective HCV NS5B inhibitors (Gopalsamy et al., "Discovery of Pyrano[3,4-b]indoles as Potent and Selective HCV NS5B Polymerase Inhibitors", J. Med. Chem., 2004, 47 (26), pp 6603-6608).

These observations led us to synthesize etodolac derivatives and to investigate their possible anticancer and anti-HCV activities. Structure of the synthesized compounds were confirmed using UV, FT-IR, ¹H-NMR, ¹³C-NMR and HR-MS spectral data besides elemental analysis and TLC.

### DETAILED DESCRIPTION OF THE INVENTION

In this study, (*R,S*)-2-[1,8-diethyl-1,3,4-tetrahydrapyrano[3,4-*b*]indol-1-yl]acetic acid (etodolac) that possess analgesic, antipyretic and antiinflammatory activity was chosen as the starting compound to design thiosemicarbazides and 1,2,4-triazole-3-thiones derivatives.

We synthesized methyl (1,8-diethyl-1,3,4,9-tetrahydropirano[3,4-*b*]indol-1-yl) acetate **SGK196** and 2-(1,8-diethyl-1,3,4,9-tetrahydropyrano[3,4-*b*]indol-1-yl)acetohydrazide **SGK197** in our laboratory. **SGK 197** and alkyl/aryl isothiocyanates were heated in ethanol to yield original 1-[2-(1,8-diethyl-1,3,4,9-tetrahydropyrano[3,4-*b*]indol-1-yl)acetyl]-4-alkyl/aryl thiosemicarbazides [**SGK224-230, SGK313**]**.** Alkaline cyclisation of the etodolac thiosemicarbazides using sodium hydroxide afforded the corresponding 5-[(1,8-diethyl-1,3,4,9-tetrahydropyrano[3,4-b]indol-1-yl)methyl]-4-substituted-2,4-dihydro-3*H*-1,2,4-triazole-3-thiones [**SGK238-244, SGK315].** Synthetic route to compounds [**SGK224-230, SGK313**] and [**SGK238-244, SGK315**] are shown in Scheme 1. Purity of the synthesized compounds in this study was confirmed by thin layer chromatography and elemental analysis. Characterization of these compounds synthesized from etodolac, were performed by the help of FT-IR, ¹H-NMR, ¹³C-NMR and HR-MS spectral data.

In the invention, the isothiocyanates which were used in the stage of obtaining **[SGK224-230, SGK313]** from **[SGK197]** are selected from the group including preferably methylisothiocyanate, ethylisothiocyanate, propylisothiocyanate, butylisothiocyanate, benzylisothiocyanate, allyl isothiocyanate, phenyl isothiocyanate and cyclohexylisothiocyanate.

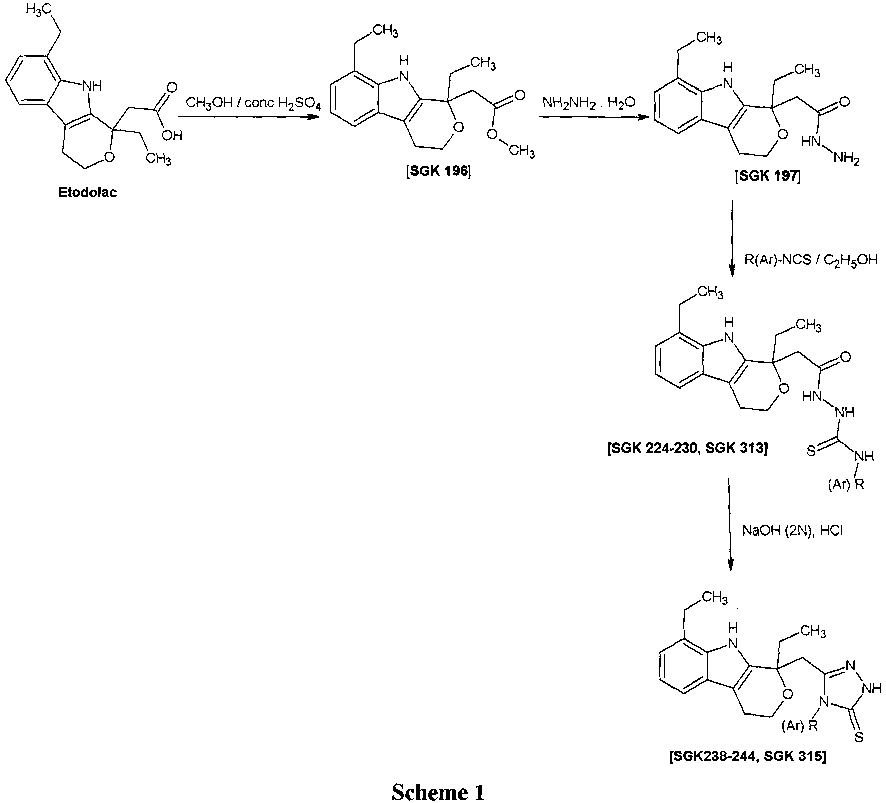

### Synthesis of methyl (1,8-diethyl-1,3,4,9-tetrahydropirano[3,4-b]indol-1-yl) acetate [SGK 196] (TR 2012/07059)

Etodolac (0.01 mol) and methanol (16 mL) refluxed for 3 h in a few drops of concentrated sulfuric acid. The contents of the flask were subsequently cooled and neutralized by using NaHCO₃ (5%). The resulting precipitate was filtered, dried and recrystallized twice from ethanol.

**Compound** [**SGK 196]**: Yield 66%, m.p. 127-130°C (m.p. 128-130°C [13, 14] ). IR (vₘₐₖₛ, cm⁻¹): 3379 (NH), 1705 (C=O). ¹H NMR (400 MHz, DMSO-d*₆*) δ ppm: 0.83 (3H, t, - CH₂-CH₃ at C₁); 1.37 (3H, t, -CH₂-CH₃ at C₈); 1.98-2.18 (2H, m, -CH₂-CH₃ at C₁); 2.73-3.04 (6H, m, -CH₂-CH₃ at C₈, -CH₂-COOCH₃ at C₁ and -CH₂ at C₄); 3.72 (3H, s, -COOCH₃); 3.94-4.06 (2H, m, -CH₂ at C₃); 7.01 (1H, d, C₇-H, *J=*6.8 Hz); 7.06 (1H, t, C₆-H); 7.36 (1H, d, C₅-H, *J*=7.2 Hz); 9.06 (s, 1H, indole N-H). ¹³C NMR (100 MHz) (DMSO-d*₆*/TMS) δ ppm: 8.26 (C-12); 14.92 (C-10); 22.34 (C-9); 24.18 (C-4); 31.18 (C-11); 42.93 (C-13); 51.68 (-COOCH₃); 60.43 (C-3); 75.82 (C-1); 107.83 (C-4a); 115.88 (C-5); 119.32 (C-6); 120.15 (C-7); 126.47 (C-5a); 127.03 (C-8); 135.01 (C-1a); 136.28 (C-8a); 170.52 (C=O). Analysis for C₁₈H₂₃NO₃ (301.380). Calcd: C, 71.73; H, 7.69; N, 4.65%. Found: C, 70.85; H, 7.24; N, 4.75%.

### Synthesis of 2-(1,8-diethyl-1,3,4,9-tetrahydropyrano[3,4-b]indol-1-yl)acetohydrazide [SGK 197] (TR 2012/07059)

To methanolic solution of compound [**SGK 196**] (20 mL, 0.01 mol) was added hydrazine-hydrate (80%, 7 mL) and refluxed for 3 h. The reaction mixture was then cooled, diluted with water and allowed to stand overnight. The precipitated solid was washed with water, dried and recrystallized twice from petroleum ether.

**Compound [SGK 197]:** Yield 80%, m.p. 186-188°C. IR (vₘₐₓ, cm⁻¹): 3354, 3310 (NH), 1651 (C=O). ¹H NMR (400 MHz, DMSO-d*₆*) δ ppm: 0.62 (3H, t, -CH₂-CH₃ at C₁); 1.26 (3H, t, -CH₂-CH₃ at C₈); 2.00-2.07 (2H, m, -CH₂-CH₃ at C₁); 2.58-2.86 (6H, m, CH₂-CONHNH₂, -CH₂-CH₃ at C₈ and -CH₂ at C₄); 3.91-3.97 (2H, m, -CH₂ at C₃); 4.27 (2H, b.s., NH-NH₂); 6.87-7.24 (3H, m, Ar-H); 8.94 (1H, s, NHNH₂); 10.55 (s, 1H, indole N-H). ¹³C-NMR(100 MHz) (DMSO-d*₆*/TMS) δ ppm: 8.29 (C-12); 14.84 (C-10); 22.42 (C-9); 24.22 (C-4); 30.85 (C-11); 42.65 (C-13); 60.29 (C-3); 75.79 (C-1); 107.40 (C-4a); 115.88 (C-6); 119.18 (C-5); 120.06 (C-7); 126.57 (C-5a); 126.94 (C-8); 134.88 (C-1a); 137.32 (C-8a); 169.21 (C=O). Analysis for C₁₇H₂₃N₃O₂ (301.383). Calcd: C, 67.75; H, 7.69; N, 13.94%. Found: C, 67.33; H, 6.95; N, 13.73%.

Etodolac methyl ester has been previously reported in the literature. This compound which was not original was synthesized using a different method in this study. Etodolac hydrazide, **SGK 197** was first reported in this study. In the ¹H NMR spectra of 2-(1,8-diethyl-1,3,4,9-tetrahydropyrano[3,4-b]indole-1-yl) acetohydrazide **SGK 197**, singlet signals derived from hydrazide structure appeared at 4.27 ppm (-NHNH₂) and 8.94 ppm (-NHNH₂) showing the integration for two protons and one proton, respectively.

### Synthesis of 1-[2-(1,8-diethyl-1,3,4,9-tetrahydropyrano[3,4-b]indol-1-yl)acetyl]-4-alkyl/aryl thiosemicarbazides [SGK224-230, SGK313]

A solution of 0.01 mol of 2-(1,8-diethyl-1,3,4,9-tetrahydropyrano[3,4-*b*]indole-1-yl)acetohydrazide **[SGK197]** and equimolar amount of appropriate isothiocyanate in 20 mL of etanol was heated under reflux for 2 h. The precipitate obtained was filtered-off, washed with water and cleaned twice with boiling etanol.

The FT-IR spectra of etodolac thiosemicarbazide derivatives displayed C=O bands at 1674-1688 cm⁻¹ and C=S bands at 1188-1217 cm⁻¹ In the ¹H-NMR spectra, all protons were observed accordingly to the expected chemical shift and integral values. For the thiosemicarbazides, the signals of the proton linked to N¹,N² and N⁴ nitrogens were shown at 10.48-10.55, 9.58-9.96 and 7.03-9.08 ppm, respectively. In addition, NH protons of compound **SGK 225** was observed to exchange with D₂O in the spectrum. All the other aliphatic and aromatic protons were observed at expected regions. In the ¹³C-NMR spectra of selected prototype compounds **SGK 224** and **SGK 225,** hydrazide C=O were observed at 170.21 and 168.95 ppm, respectively. The peaks resonated at 183.79 and 170.52 ppm in the ¹³C-NMR spectra of these compounds, assigned for C=S, confirming thione form of thiosemicarbazides The EI-MS of the selected compound **SGK 225** displayed molecular ion at m/z 419 which confirmed its molecular weight. The major fragmentation pattern involved the cleavage of the CONH-NH-CS bonds of amide.

### Synthesis of 5-[(1,8-diethyl-1,3,4,9-tetrahydropyrano[3,4-b]indol-1-yl)methyl]4-substituted -2,4-dihydro-3H-1,2,4-triazole-3-thiones [SGK238-244, SGK315]

A solution of 0.01 mol compounds **SGK224-230, SGK313** in 2N sodium hydroxide solution (25 mL) was heated under reflux for 4 h. After cooling to room temperature, concentrated hydrochloric acid was added. The precipitate was filtered and washed several times with distilled water. The compounds were recrystallized from ethanol.

Alkaline cyclisation of etodolac thiosemicarbazides [**SGK224-230, SGK313**] using sodium hydroxide afforded the corresponding 5-[(1,8-diethyl-1,3,4,9-tetrahydropyrano[3,4-b]indol-1-yl)methyl]-4-substituted-2,4-dihydro-3H-1,2,4-triazole-3-thiones [**SGK238-244, SGK315**]. Etodolac triazoles were characterized by their melting points and spectral data. Formation of triazoles [**SGK238-244, SGK315**] was confirmed by IR spectroscopy, the existence of triazoles in thione form was also confirmed by the absence of SH stretching in the characteristic region of 2550 cm⁻¹ and presence of C=S stretching in the region of 1215-1262 cm⁻¹. The cyclization of thiosemicarbazides [**SGK224-230, SGK313**] to the products [**SGK238-244, SGK315**] was indicated in the IR spectra by appearance of a single absorption for NH and disapperance of the carbonyl absorption. The formation of triazole was confirmed in ¹H-NMR spectra by the apperance of only one relatively broad NH proton signal in the region of 13.38-13.82 ppm, whereas S-H proton at 1.0-3.7 ppm was not detected and so their thionic form was proved. Triazole NH resonances were observed to be shifted to downfield because of strong intermolecular hydrogen bonding as previously reported. Remaining chemical shifts were also recorded at expected values. The ¹³C NMR spectra of compounds **SGK239, SGK243** and **SGK244** which were chosen as prototypes, verified the proposed 1,2,4-triazole-3-thione structure. In ¹³C-NMR spectra, the absence of the signal for C=O and apperance of the signal for C=N respectively, 148.83, 150.46 and 154.31 ppm also confirmed the formation of the 1,2,4-triazoles. The -C=S form is present also in dimethyl sulfoxide, as suggested by the respective ¹³C-NMR spectral data. The presence of the signals at 165.70, 167.82 and 170.66 ppm respectively for selected compounds, characteristic for the C=S group, indicated that these compounds from the 1,2,4-triazole class existed in solution predominantly in the thione form. High resolution mass spectra (HR-MS) confirmed the molecular weights, empirical formulae of compounds, **SGK238**, **SGK243**, **SGK244** and 1,2,4-triazoles and fragments, with less than 5 mmu bias between calculated and experimental m/z values of either molecular or fragment ions. Fragmentation pattern for all etodolac 1,2,4-triazoles supported the proposed structures. The fragment ion peaks especially [M-N₂]⁺, [M-HCNS]⁺, [M-RNCS]⁺ and [M-S]⁺ are the diagnostic peaks for 1,2,4-triazoles rings.

The invention which was mentioned here is essentially the synthetic method for 1-[2-(1,8-diethyl-1,3,4,9-tetrahydropyrano[3,4-b]indol-1-yl)acetyl]-4-alkyl/arylthiosemicarbazide [SGK224-230, SGK313] compounds and it is characterised in that 2-(1,8-diethyl-1,3,4,9-tetrahydropyrano[3,4-b]indole-1-yl)acetohydrazide [SGK197] is synthesised by substituted isothiocyanates with at least 60-97% yield and it is a synthetic method for 5-[(1,8-diethyl-1,3,4,9-tetrahydropyrano[3,4-b]indole-1-yl)methyl]-4-substituted-2,4-dihydro-3H-1,2,4-triazole-3-thione [SGK238-244, SGK315] compounds and it is also characterised in that it is synthesised by the reaction of 1-[2-(1,8-diethyl-1,3,4,9-tetrahydropyrano[3,4-b]indole-1-yl)acetyl]-4-alkyl/arylthiosemicarbazide [SGK224-230, SGK313] compounds in the alkaline medium with at least 50-80% yield.
In addition, the invention which was mentioned here consists of the synthesis of the following compounds and it is also not limited to them;
**SGK224** 1-[2-(1,8-diethyl-1,3,4,9-tetrahydropyrano[3,4-b]indol-1-yl)acetyl]-4-methyl thiosemicarbazide
**SGK225** 1-[2-(1,8-Diethyl-1,3,4,9-tetrahydropyrano[3,4-b]indol-1-yl)acetyl]-4-ethyl thiosemicarbazide
**SGK226** 1-[2-(1,8-Diethyl-1,3,4,9-tetrahydropyrano[3,4-b]indole-1-yl)acetyl]-4-propyl thiosemicarbazide
**SGK227** 1-[2-(1,8-Diethyl-1,3,4,9-tetrahydropyrano[3,4-b]indol-1-yl)acetyl]-4-butyl thiosemicarbazide
**SGK228** 1-[2-(1,8-Diethyl-1,3,4,9-tetrahydropyrano[3,4-b]indol-1-yl)acetyl]-4-benzyl thiosemicarbazide
**SGK229** 1-[2-(1,8-Diethyl-1,3,4,9-tetrahydropyrano[3,4-b]indole-1-yl)acetyl]-4-allyl thiosemicarbazide
**SGK230** 1-[2-(1,8-Diethyl-1,3,4,9-tetrahydropyrano[3,4-b]indol-1-yl)acetyl]-4-phenyl thiosemicarbazide
**SGK313** 1-[2-(1,8-Diethyl-1,3,4,9-tetrahydropyrano[3,4-b]indol-1-yl)acetyl]-4-cyclohexyl thiosemicarbazide
**SGK238** 5-[(1,8-Diethyl-1,3,4,9-tetrahydropyrano[3,4-b]indol-1-yl)methyl]-4-methyl-2,4-dihydro-3H-1,2,4-triazole-3-thione
**SGK239** 5-[(1,8-Diethyl-1,3,4,9-tetrahydropyrano[3,4-b]indol-1-yl) methyl]-4-ethyl-2,4-dihydro-3H-1,2,4-triazole-3-thione
**SGK240** 5-[(1,8-Diethyl-1,3,4,9-tetrahydropyrano[3,4-b]indol-1-yl)methyl]-4-propyl-2,4-dihydro-3H-1,2,4-triazole-3-thione
**SGK241** 5-[(1,8-Diethyl-1,3,4,9-tetrahydropyrano[3,4-b]indol-1-yl)methyl]-4-butyl-2,4-dihydro-3H-1,2,4-triazole-3-thione
**SGK242** 5-[(1,8-Diethyl-1,3,4,9-tetrahydropyrano[3,4-b]indol-1-yl)methyl]-4-benzyl-2,4-dihydro-3H-1,2,4-triazole-3-thione
**SGK243** 5-[(1,8-Diethyl-1,3,4,9-tetrahydropyrano[3,4-b]indol-1-yl)methyl]-4-allyl-2,4-dihydro-3H-1,2,4-triazole-3-thione
**SGK244** 5-[(1,8-Diethyl-1,3,4,9-tetrahydropyrano[3,4-b]indol-1-yl)methyl]-4-phenyl-2,4-dihydro-3H-1,2,4-triazole-3-thione
**SGK315** 5-[(1,8-Diethyl-1,3,4,9-tetrahydropyrano[3,4-b]indol-1-yl)methyl]-4-cyclohexyl-2,4-dihydro-3H-1,2,4-triazole-3-thione

| **Table 1** | | |
|---|---|---|
| ***Comp.*** | **FT-IR vₘₐₖₛ** (**cm⁻¹**) | **¹H-NMR (DMSO-d*₆*/TMS)** |
| **SGK224** | 3343, 3215 (NH); 1674 (C=O); 1198 (C=S) | 0.65 (t, 3H, -CH₂-CH₃ at C₁);_1.26 (t, 3H,-CH₂-CH₃, at C₈); 1.87- 2.04 (m, 2H, -CH₂-CH₃ at C₁); 2.66-2.88 (m, 9H, -CH₂-CO-NH at C₁, -CH₂-CH₃ at C₈, -CH₂ at C₄ and NH-CH₃); 3.97-4.00 (m, 2H, -CH₂ at C₃); 6.88-7.26 (m, 3H, Ar-H); 7.34 (b.s, 1H, N⁴-H); 9.35 (s, 1H, indole N-H); 9.58 (s, 1H, N²-H); 10.48 (s, 1H, N¹-H) |
| **SGK225** | 3377, 3308 (NH); 1674 (C=O); 1217 (C=S) | 0.62 (t, 3H, -CH₂-CH₃ at C₁); 1.02-1.08 (m, 3H, -CH₂-CH₃ at C₈), 2.02-2.06 (m, 2H, -CH₂-CH₃ at C₁); 1.26 (t, 3H, NH-CH₂-CH₃), 2.66-2.87 (m, 6H, -CH₂-CH₃ at C₈, -CH₂-CO-NH at C₁ and -CH₂ at C₄), 2.95-3.56 (m, 2H, NH-CH₂-CH₃); 3.99-4.02 (m, 2H, -CH₂ at C₃), 6.89-7.26 (m, 3H, Ar-H), 7.31 (b.s, 1H, N⁴-H), 9.35 (s, 1H, indole N-H), 9.65 (s, 1H, N²-H), 10.51 (s, 1H, N¹-H) |
| **SGK226** | 3289 (NH); 1674 (C=O); 1211 (C=S) | 0.61 (t, 3H, -CH₂-CH₃ at C₁); 0.83 (t, 3H, -CH₂-CH₃ at C₈); 1.25 (t, 3H, NH-CH₂-CH₂-CH₃); 1.44-1.47 (m, 2H, -CH₂-CH₃ at C₁); 1.94-2.04 (m, 2H, NH-CH₂-CH₂-CH₃); 2.66-2.87 (m, 6H, -CH₂-CONH at C₁, -CH₂-CH₃ at C₈ and -CH₂ at C₄); 3.17-3.38 (m, 2H, NH-CH₂-CH₂-CH₃); 3.92-4.00 (m, 2H,-CH₂ at C₃); 6.88-7.26 (m, 3H, Ar-H); 7.27 (b.s, 1H, N⁴-H); 9.38 (s, 1H, indole N-H); 9.69(s, 1H, N²-H); 10.53 (s, 1H, N¹-H) |
| **SGK227** | 3331,3215 (NH); 1674 (C=O); 1205 (C=S) | 0.61 (t, 3H, -CH₂-CH₃ at C₁); 0.87 (t, 3H, -CH₂-CH₃ at C₈); 1.24-1.29 (m, 5H, NH-CH₂-CH₂-CH₂-CH₃ and -CH₂*-*CH₃ at C₁); 1.39-1.43 (m, 2H, NH-CH₂-CH₂-CH₂-CH₃); 1.93-2.04 (m, 2H, NH-CH₂-CH₂-CH₂-CH₃); 2.68-3.53 (m, 8H, -CH₂-CH₃ at C₈, -CH₂-CONH at C₁, -CH₂ at C₄ and NH-CH₂-CH₂-CH₂-CH₃); 3.90-4.01 (m, 2H,-CH₂ at C₃); 6.89-7.25 (m, 3H, Ar-H); 7.26 (1H, b.s, N⁴-H); 9.37 (s, 1H, indole N-H); 9.68 (s, 1H, N²-H); 10.52 (s, 1H, N^{l}-H) |
| **SGK228** | 3330, 3233 (NH); 1675 (C=O); 1188 (C=S) | 0.54 (t, 3H, -CH₂-CH₃ at C₁); 1.24 (t, 3H, -CH₂-CH₃ at C₈); 1.92-2.00 (m, 2H, -CH₂-CH₃ at C₁); 2.53-2.58 (m, 2H, -CH₂-CH₃ at C₈); 2.73-2.85 (m, 4H, -CH₂-CONH at and -CH₂ at C₄); 3.73-3.84 (m, 2H, NH-CH₂-); 4.65-4.73 (m, 2H,-CH₂ at C₃); 6.87-7.33 (m, 8H, Ar-H); 7.85 (b.s, 1H, N⁴-H); 9.56 (s, 1H, indole N-H); 9.76 (s, 1H, N²-H), 10.49 (s, 1H, N¹-H) |
| **SGK229** | 3287 (NH); 1674 (C=O); 1211 (C=S) | 0.62 (t, 3H, -CH₂-CH₃ at C₁); 1.25 (t, 3H, CH₂-CH₃ at C₈); 1.96-2.03 (m, 2H, -CH₂-CH₃ at C₁); 2.65-2.69 (m, 2H, -CH₂-CH₃ at C₈); 2.74-2.87 (m, 4H, -CH₂-CONH at C₁ and -CH₂ at C₄); 3.98-4.18 (m, 4H, NH-CH₂-CH=CH₂ and -CH₂ at C₃); 5.04-5.07 (d, 1H, NH-CH₂-CH=CH₂, *J*=10.3 Hz, cis); 5.12-5.17 (d, 1H, NH-CH₂-CH=CH₂, J=17.2 Hz, trans); 5.72- 5.87 (m, 1H, NH-CH₂-CH=CH₂); 6.88-7.25 (m, 3H, Ar-H); 7.50 (b.s, 1H, N⁴-H); 9.49 (s, 1H, indole N-H); 9.72 (s, 1H, N²-H); 10.51 . (s, 1H, N¹-H) |
| **SGK230** | 3339, 3283 (NH); 1688 (C=O); 1211 (C=S) | 0.62 (t, 3H, -CH₂-CH₃ at C₁); 1.26 (t, 3H, -CH₂-CH₃ at C₈); 2.05-2.08 (m, 2H, -CH₂-CH₃ at C₁); 2.65-2.69 (m, 2H, -CH₂-CH₃ at C₈); 2.80-3.42 (m, 4H, -CH₂-CONH at and -CH₂ at C₄); 3.99-4.02 (m, 2H, -CH₂ at C₃); 6.88-7.53 (m, 8H, Ar-H); 9.08 (b.s, 1H, N⁴-H); 9.84 (s, 1H, indole N-H); 9.96 (s, 1H, N²-H); 10.52 (s, 1H, N¹-H) |
| **SGK313** | 3275 (NH); 1676 (C=O); 1213 (C=S) | 0.58 (t, 3H, -CH₂-CH₃ at C₁); 1.06-1.16 (m, 3H, -CH₂-CH₃ at C₈); 1.23-2.05 (m, 13H, -CH₂-CH₃ at C₁ and C₆H₁₁); 2.68-2.87 (m, 6H, -CH₂-CH₃ at C₈, -CH₂-CONH at C₁ and -CH₂ at C₄); 3.92-4.06 (m, 2H,-CH₂ at C₃); 6.88-7.26 (m, 3H, Ar-H); 7.03 (b.s, 1H, N⁴-H); 9.41 (s, 1H, indole N-H); 9.75 (s, 1H, N²-H), 10.55 (s, 1H, N¹-H) |
| **SGK238** | 3279 (NH); 1239 (C=S) | 0.64 (t, 3H, -CH₂-CH₃ at C₁); 1.27 (t, 3H, -CH₂-CH₃ at C₈); 1.67-2.09 (m, 2H, -CH₂-CH₃ at C₁); 2.57-2.89 (m, 4H, -CH₂ at C₁and -CH₂-CH₃ at C₈); 3.30-3.46 (m, 5H, -CH₂ at C₄ and N-CH₃); 3.85-3.97 (m, 2H-CH₂ at C₃); 6.89-7.24 (m, 3H, Ar-H); 10.67 (s, 1H, indole N-H); 13.49 (s, 1H, triazole N-H) |
| **SGK239** | 3340,3239 (NH); 1215 (C=S) | 0.66 (m, 3H, -CH₂-CH₃ at C₁); 1.21-1.30 (m, 6H, -CH₂-CH₃ at C₈ and -N-CH₂-CH₃); 1.72-2.10 (m, 2H, -CH₂-CH₃ at C₁); 2.53-2.68 (m, 2H, -CH₂-CH₃ at C₈); 2.84-3.46 (m, 4H, -CH₂ at C₁ and -CH₂ at C₄); 3.80-4.07 (m, 4H, -CH₂ at C₃ ve -N-CH₂-CH₃); 6.89-7.24 (m, 3H, Ar-H); 10.68 (s, 1H, indole N-H); 13.45 (s, 1H, triazole N-H) |
| **SGK240** | 3341, 3160 (NH); 1251 (C=S) | 0.65 (t, 3H, -CH₂-CH₃ at C₁); 0.86 (t, 3H, -CH₂-CH at C₈); 1.28 (t, 3H, N-CH₂-CH₂-CH₃); 1.64-2.10 (m, 4H, -CH₂-CH₃ at C₁ and C₈); 2.54-2.65 (m, 2H, N-CH₂-CH₂-CH₃); 2.84- 3.46 (m, 4H, -CH₂ at C₁ and -CH₂ at C₄); 3.78-3.88 (m, 2H, N-CH₂-CH₂-CH₃); 3.90-3.97 (m, 2H, -CH₂ at C₃); 6.89-7.24 (m, 3H, Ar-H); 10.69 (s, 1H, indole N-H); 13.49 (s, 1H, triazole N-H). |
| **SGK241** | 3329, 3251 (NH); 1262 (C=S) | 0.66 (t, 3H, -CH₂-CH₃ at C₁); 0.81-1.31 (m, 10H, -CH₂-CH₃ at C₈, N-CH₂-CH₂-CH₂-CH₃); 1.58-1.66 (m, 2H, -CH₂-CH₃ at C₁); 1.73-2.68 (m, 4H, -CH₂ at C₁ and -CH₂-CH₃ at C₈); 2.84-2.89 (m, 2H, - CH₂ at C₄); 3.79-3.97 (m, 4H, N-CH₂-CH₂-CH₂-CH₃ and -CH₂ at C₃); 6.89-7.24 (m, 3H, Ar-H); 10.71 (s, 1H, indole N-H); 13.55 (b.s, 1H, triazole N-H). |
| **SGK242** | 3270 (NH); 1261 (C=S) | 0.67 (t, 3H, -CH₂-CH₃ at C₁); 1.26 (t, 3H, -CH₂-CH₃ at C₈); 1.81-2.05 (m, 2H, -CH₂-CH₃ at C₁); 2.82-3.35 (m, 6H, -CH₂-CH₃ at C₈, -CH₂- at C₁ and -CH₂ at C₄); 3.60-3.88 (m, 2H, -CH₂ at C₃); 5.21-5.32 (m, 2H, N-CH₂); 6.89-7.36 (m, 8H, Ar-H); 10.62 (s, 1H, indole N-H); 13.67 (s, 1H, triazole N-H) |
| **SGK243** | 3349, 3258 (NH); 1251 (C=S) | 0.66-0.86 (m, 3H, -CH₂-CH₃ at C₁); 1.27 (t, 3H, -CH₂-CH₃ at C₈); 1.65-1.95 (m, 2H, -CH₂-CH₃ as C₁); 2.57-3.32 (m, 6H, -CH₂-CH₃ at C₈, -CH₂- at C₁ and -CH₂- at C₄); 3.66-3.86 (m, 2H, -CH₂- at C₃); 4.53-4.86 (2H, m, -CH₂-CH=CH₂); 4.98 (d, 1H, -CH₂-CH=CH₂, *J*=17.25 Hz, *trans*); 5.18 (d, 1H, CH₂-CH=CH₂, *J*=10.41 Hz, *cis*); 5.72-6.07 (m, 1H, -CH₂-CH=CH₂); 6.90-7.24 (m, 3H, Ar-H); 10.68 (s, 1H, indole N-H); 13.54 (s, 1H, triazole N-H). |
| **SGK244** | 3336 (NH); 1232 (C=S) | 0.47 (t, 3H, CH₂-CH₃ at C₁); 1.24 (t, 3H, CH₂-CH₃ at C₈); 1.71-2.02 (m, 2H, -CH₂-CH₃ at C₁); 2.29-2.47 (m, 2H, -CH₂-CH₃ at C₈); 2.79-3.21 (m, 6H, -CH₂-, at C₁,-CH₂ at C₃ and C₄); 6.87-7.51 (m, 8H, Ar-H); 10.56 (s, 1H, indole N-H); 13.82 (b.s, 1H, triazole N-H). |
| **SGK315** | 3254 (NH); 1240 (C=S) | 0.64-0.74 (m, 3H, -CH₂-CH₃ at C₁); 0.85-1.25 (m, 3H, -CH₂-CH₃ at C₈); 1.27-3.91 (m, 19H, CH₂-CH₃ at C₁ and C₈; -CH₂- at C₁ -CH₂ at C₄, and C₆H₁₁); 3.98-4.19 (m, 2H, -CH₂ at C₃); 6.95-7.41 (m, 3H, Ar-H); 10.89 (s, 1H, indole N-H); 13.38 (b.s, 1H, triazole NH) |
| SGK224: **¹³C**-**NMR** (DMSO-*d₆*/TMS): 8.33 (C-12); 14.98 (C-10); 22.43 (C-9); 24.35 (C-4); 31.20 (C-11); 31.79 (NH-CH₃); 43.07 (C-13); 61.06 (C-3); 76.57 (C-1); 108.21 (C-4a); 116.64 (C-6); 119.99 (C-5); 120.95 (C-7); 127.22 (C-5a); 127.84 (C-8); 135.79 (C-1a); 137.44 (C-8a); 170.21 (C=O); 183.79 (C=S). | | |
| **SGK225: ¹³C-NMR (DMSO-d*₆*/TMS)**:8,26(C-12);14,67(NH-CH₂-CH₃);14.91 (C-10); 22.28 (C-9); 24.52 (C-4); 30.93 (C-l1); 42.70 (C-13); 51.84 (NH-CH₂-CH₃); 60.81 (C-3); 76.32 (C-1); 107.61 (C-4a); 115.94 (C-6); 119.30 (C-5); 120.26 (C-7); 126.37 (C-5a); 127.09 (C-8); 134.97 (C-1a); 136.35 (C-8a); 168.95 (C=O); 170.52 (C=S). | | |
| **SGK239**: **¹³C-NMR (DMSO-d*₆*/TMS):** 7.68 (C-12); 13.15 (N-CH₂-CH₃); 14.49 (C-10); 21.79 (C-9); 23.75 (C-4); 31.09 (C-11); 33.48 (C-13); 38.42 (N-CH₂-CH₃); 60.27 (C-3); 76.44 (C-1); 107.99 (C-4a); 115.41 (C-6); 118.73 (C-5); 119.77 (C-7); 125.84 (C-5a); 126.63 (C-8); 134.63 (C-1a); 135.17 (C-8a); 148.83 (triazol -C=N); 165.70 (C=S) | | |
| **SGK243: ¹³C-NMR (DMSO-d₆/TMS)**:8.22 (C-12); 15.08 (C-10); 22.42 (C-9); 24.39 (C-4); 31.70 (C-11); 34.41 (C-13); 45.67 (N-CH₂-CH=CH₂); 61.23 (C-3); 77.52 (C-1); 109.04 (C-4a); 116.61 (-CH₂-CH=CH₂); 118.25 (C-6); 120.01 (C-5); 121.02 (C-7); 127.08 (C-5a); 127.95 (C-8); 133.26 (-CH₂-CH=CH₂); 135.95 (C-1a); 136.43 (C-8a); 150.46 (triazole C=N); 167.82 (C=S) | | |
| **SGK244**: **¹³C-NMR (DMSO-d₆/TMS)**: 7.54 (C-12); 14.41 (C-10); 21.72 (C-9); 23.67 (C-4); 31.07 (C-11); 34.92 (C-13); 59.50 (C-3); 76.26 (C-1); 107.87 (C-4a); 115.34 (C-6); 119.28 (C-5); 121.37 (C-7); 125.65 (C-5a); 126.59 (C-2' ve C-6'); 127.05 (C-4'); 129.27 (C-3' ve C-5'); 132.77 (C-8); 134.26 (C-1'); 134.58 (C-1a); 135.73 (C-8a); 154.31 (triazole C=N); 170.66 (C=S) | | |

| **Table 2** | | | | | | | | | | | | | |
|---|---|---|---|---|---|---|---|---|---|---|---|---|---|
| **Comp.** | **Ar** | **Molecular Formula** | **M.W (g/mol)** | **Yield** | **M.p. (°C)** | **Elementel Analysis** | | | | | | | |
| | | | | | | **Calculated (%)** | | | | **Found (%)** | | | |
| | | | | | | **C** | **H** | **N** | **S** | **C** | **H** | **N** | **S** |
| **SGK224** | -CH₃ | C₁₉H₂₆N₄O₂S.1/2 H₂O | 383.527 | 60 | 208-211 | 60.94 | 7.00 | 14.96 | 8.56 | 59.50 | 7.09 | 14.60 | 8.36 |
| **SGK225** | -CH₂CH₃ | C₂₀H₂₈N₄O₂S | 388.527 | 82 | 215 | 61.83 | 7.26 | 14.42 | 8.25 | 61.73 | 7.00 | 13.94 | 7.65 |
| **SGK226** | -CH₂CH₂CH₃ | C₂₁H₃₀N₄O₂S | 402.554 | 84 | 225 | 62.66 | 7.51 | 13.92 | 7.97 | 62.91 | 7.38 | 13.80 | 7.98 |
| **SGK227 £** | -CH₂CH₂CH₂ CH₃ | C₂₂H₃₂N₄O₂S | 416.580 | 71 | 180-181 | 63.43 | 7.74 | 13.45 | 7.70 | 63.91 | 7.58 | 13.49 | 7.29 |
| **SGK228** | -CH₂-C₆H₅ | C₂₅H₃₀N₄O₂S | 450.596 | 83 | 148-150 | 66.64 | 6.71 | 12.43 | 7.12 | 65.87 | 6.67 | 11.98 | 7.63 |
| **SGK229** | CH₂-CH=CH₂ | C₂₁H₂₈N₄O₂S | 400.538 | 72 | 213 | 62.97 | 7.05 | 13.99 | 8.01 | 63.08 | 6.87 | 13.97 | 8.09 |
| **SGK230** | -C₆H₅ | C₂₄H₂₈N₄O₂S | 436.570 | 97 | 164 | 66.03 | 6.46 | 12.83 | 7.34 | 65.53 | 6.21 | 12.34 | 7.00 |
| **SGK313** | -C₆H₁₁ | C₂₄H₃₄N₄O₂S | 442.617 | 63 | 205 | 65.13 | 7.74 | 12.66 | 7.24 | 65.38 | 7.63 | 12.59 | 7.69 |

| **Table 3** | | | | | | |
|---|---|---|---|---|---|---|
| **Comp** | **Ar** | **Yield (%)** | **Molecular Formula** | **M.W. (g/mol)** | **M.p. (°C)** | **HR-MS (EI⁺) Calculated/Found (m/z)** |
| **SGK238** | -CH₃ | 71 | C₁₉H₂₄N₄OS | 356.485 | 227-229 | 356.1670/356.1663 (M⁺) (C₁₉H₂₄N₄OS); 341.1441/341.0010; 328.1609/328.1317; 324.1950/324.1943 (C₁₉H₂₄N₄O); 297.1841/297.9783; 281.1581/281.1633; 279.1372/279.1453; 269.1648/269.9906; 268.1576/268.1513; 243.1623/243.1576; 229.1466/229.1409; 228.1382/228.1345 (C₁₅H₁₈NO); 227.1310/227.1298 |
| **SGK239** | -CH₂CH₃ | 82 | C₂₀H₂₆N₄OS | 370.512 | 159-160 | 370.1827/370.1821(M⁺) (C₂₀H₂₆N₄OS); 341.1682/341.1605; 341.1441/341.1382 (C₁₈H₂₁N₄OS); 229.1466/ 229.1399; 228.1382/228.1389 (C₁₅H₁₈NO); 227.1310/227.1399 |
| **SGK240** | -CH₂CH₂CH₃ | 82 | C₂₄H₂₈N₄OS | 384.538 | 138-140 | 384.1984/384.1990 (M⁺) (C₂₁H₂₈N₄OS); 355.1839/355.1574; 355.1587/355.1574; 324.2070/324.9914; 229.1466/229.1416; 228.1382/228.1369 (C₁₅H₁₈NO); 227.1310/227.1302 |
| **SGK241** | -CH₂CH₂CH₂CH₃ | 78 | C₂₂H₃₀N₄OS | 398.565 | 186-188 | 398.2140/398.2113 (M⁺) (C₂₂H₃₀N₄OS); 397.2057/397.2112; 369.1995/369.1689; 369.1743/369.1689; 229.1466/229.1439; 228.1382/228.1374 (C₁₅H₁₈NO); 227.1310/227.1292 |
| **SGK242** | -CH₂-C₆H₅ | 50 | C₂₅H₂₈N₄OS | 432.581 | 163-166 | 432.1983/432.1975 (M⁺) (C₂₅H₂₈N₄OS); 403.1839/403.1498; 269.1648/269.9848; 229.1466/229.1417; 228.1382/228.1385 (C₁₅H₁₈NO); 227.1310/227.1302 |
| **SGK243** | -CH₂-CH=CH₂ | 62 | C₂₁H₂₆N₄OS | 382.522 | 133-135 | 382.1827/382.1849 (M⁺ (C₂₁H₂₆N₄OS); 353.1682/353.1339; 352.1609/352.2905; 350.2107/350.2144 (C₂₁H₂₆N₄O); 340.1358/340.9752; 323.1998/323.9792; 321.1841/321.1769; 281.1528/281.9843; 279.1372/279.2179; 269.1648/269.9821; 243.1623/243.9860; 229.1466/229.1409; 228.1382/228.1344 (C₁₅H₁₈NO); 227.1310/227.1912 |
| **SGK244** | -C₆H₅ | 81 | C₂₄H₂₆N₄OS | 418.554 | 277-280 | 418.1827/418.1790 (M⁺) (C₂₄H₂₆N₄OS); 390.1765/390.1391; 389.1431/389.1441 (C₂₂H₂₁N₄OS); 386.2106/386.2105; 362.1201/362.9946; 355.1685/355.0124; 340.1358/340.9767; 281.1528/281.9729; 229.1466/229.1409; 228.1382/228.1376 (C₁₅H₁₈NO); 227.1310/227.1298; 227.1057/227.1281 |
| **SGK315** | -C₆H₁₁ | 80 | C₂₄H₃₂N₄OS | 424.602 | 157-160 | 424.2296/424.2266 (M⁺) (C₂₄H₃₂N₄OS); 392.2576/392.2709; 392.1922/392.2621; 362.2227/362.9829; 341.1441/341.1542; 281.1528/281.9915; 243.1623/243.9850; 229.1466/229.1490; 228.1382/228.1379 (C₁₅H₁₈NO); 227.1310/227.1393 |

### COMPOUNDS

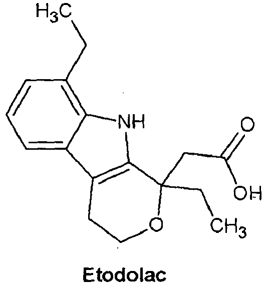

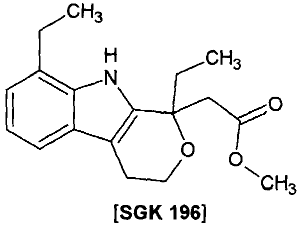

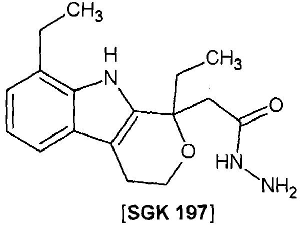

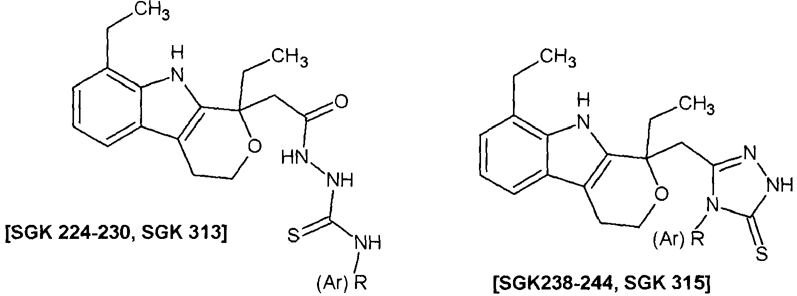

## Claims

1. A compound selected from one of the group consisting of:
1-[2-(1,8-diethyl-1,3,4,9-tetrahydropyrano[3,4-*b*]indole-1-yl)acetyl]-4-methyl thiosemicarbazide,
1-[2-(1,8-diethyl-1,3,4,9-tetrahydropyrano[3,4-*b*]indole-1-yl)acetyl]-4-ethyl thiosemicarbazide,
1-[2-(1,8-diethyl-1,3,4,9-tetrahydropyrano[3,4-*b*]indole-1-yl)acetyl]-4-propyl thiosemicarbazide,
1-[2-(1,8-diethyl-1,3,4,9-tetrahydropyrano[3,4-*b*]indole-1-yl)acetyl]-4-butyl thiosemicarbazide,
1-[2-(1,8-diethyl-1,3,4,9-tetrahydropyrano[3,4-*b*]indole-1-yl)acetyl]-4-benzyl thiosemicarbazide,
1-[2-(1,8-diethyl-1,3,4,9-tetrahydropyrano[3,4-*b*]indole-1-yl)acetyl]-4-allyl thiosemicarbazide,
1-[2-(1,8-diethyl-1,3,4,9-tetrahydropyrano[3,4-*b*]indole-1-yl)acetyl]-4-phenyl thiosemicarbazide, and
1-[2-(1,8-diethyl-1,3,4,9-tetrahydropyrano[3,4-*b*]indole-1-yl)acetyl]-4-cyclohexyl thiosemicarbazide.

2. A synthesis method for producing 1-[2-(1,8-diethyl-1,3,4,9-tetrahydropyrano[3,4-*b*]indole-1-yl)acetyl]-4-alkyl/aryl-thiosemicarbazide compounds of claim 1 **characterised in that** 2-(1,8-diethyl-1,3,4,9-tetrahydropyrano[3,4-*b*]indole-1-yl) acetohydrazide is reacted with substituted isothiocyanates in an ethanolic medium.

3. A compound selected from one of the group consisting of:
5-[(1,8-diethyl-1,3,4,9-tetrahydropyrano[3,4-*b*]indol-1-yl)methyl]-4-methyl-2,4-dihydro-3H-1,2,4-triazole-3-thione,
5-[(1,8-diethyl-1,3,4,9-tetrahydropyrano[3,4-*b*]indol-1-yl)methyl]-4-ethyl-2,4-dihydro-3H-1,2,4-triazole-3-thione,
5-[(1,8-diethyl-1,3,4,9-tetrahydropyrano[3,4-*b*]indol-1-yl)methyl]-4-propyl-2,4-dihydro-3H-1,2,4-triazole-3-thione,
5-[(1,8-diethyl-1,3,4,9-tetrahydropyrano[3,4-*b*]indol-1-yl)methyl]-4-butyl-2,4-dihydro-3H-1,2,4-triazole-3-thione,
5-[(1,8-diethyl-1,3,4,9-tetrahydropyrano[3,4-*b*]indol-1-yl)methyl]-4-benzyl-2,4-dihydro-3H-1,2,4-triazole-3-thione,
5-[(1,8-diethyl-1,3,4,9-tetrahydropyrano[3,4-*b*]indol-1-yl)methyl]-4-allyl-2,4-dihydro-3H-1,2,4-triazole-3-thione,
5-[(1,8-diethyl-1,3,4,9-tetrahydropyrano[3,4-*b*]indol-1-yl)methyl]-4-phenyl-2,4-dihydro-3H-1,2,4-triazole-3-thione, and
5-[(1,8-diethyl-1,3,4,9-tetrahydropyrano[3,4-*b*]indol-1-yl)methyl]-4-cyclohexyl-2,4-dihydro-3H-1,2,4-triazole-3-thione.

4. A synthesis method for producing 5-[(1,8-diethyl-1,3,4,9-tetrahydropyrano[3,4-*b*]indole-1-yl)methyl]-4-substituted-2,4-dihydro-3H-1,2,4-triazole-3-thione compounds of claim 3 **characterised in that** said compounds are synthesised by the reaction of 1-[2-(1,8-diethyl-1,3,4,9-tetrahydropyrano[3,4-b]indole-1-yl)acetyl]-4-alkyl/aryl thiosemicarbazide compounds where alkyl/aryl group is selected from one of the group of methyl, ethyl, propyl, butyl, benzyl, allyl, phenyl and cyclohexyl; in an alkaline medium followed by neutralization with an acid.

## Patentansprüche

1. Eine Zusammensetzung ausgewählt aus der Gruppe bestehend aus:
1-[2-(1,8-diethyl-1,3,4,9-tetrahydropyrano[3,4-*b*]indol-1-yl)acetyl]-4-methyl thiosemicarbazid,
1-[2-(1,8-diethyl-1,3,4,9-tetrahydropyrano[3,4-*b*]indol-1-yl)acetyl]-4-ethyl thiosemicarbazid,
1-[2-(1,8-diethyl-1,3,4,9-tetrahydropyrano[3,4-*b*]indol-1-yl)acetyl]-4-propyl thiosemicarbazid,
1-[2-(1,8-diethyl-1,3,4,9-tetrahydropyrano[3,4-*b*]indol-1-yl)acetyl]-4-butyl thiosemicarbazid,
1-[2-(1,8-diethyl-1,3,4,9-tetrahydropyrano[3,4-*b*]indol-1-yl)acetyl]-4-benzyl thiosemicarbazid,
1-[2-(1,8-diethyl-1,3,4,9-tetrahydropyrano[3,4-*b*]indol-1-yl)acetyl]-4-allyl thiosemicarbazid,
1-[2-(1,8-diethyl-1,3,4,9-tetrahydropyrano[3,4-*b*]indol-1-yl)acetyl]-4-phenyl thiosemicarbazid, und
1-[2-(1,8-diethyl-1,3,4,9-tetrahydropyrano[3,4-*b*]indol-1-yl)acetyl]-4-cyclohexyl thiosemicarbazid.

2. Eine Synthese Methode zur Herstellung von 1-[2-(1,8-diethyl-1,3,4,9-tetrahydropyrano[3,4-*b*] indol-1-yl)acetyl]-4-alkyl/aryl-thiosemicarbazid Zusammensetzungen gemäß Anspruch 1, **dadurch gekennzeichnet, dass** 2-(1,8-diethyl-1,3,4,9-tetrahydropyrano[3,4-*b*]indol-1-yl) acetohydrazid mit substituiertem Isothiocyanat in einem ethanolischen Medium umgesetzt wird.

3. Eine Zusammensetzung ausgewählt aus der Gruppe bestehend aus:
5-[(1,8-diethyl-1,3,4,9-tetrahydropyrano[3,4-*b*]indol-1-yl)methyl]-4-methyl-2,4-dihydro-3H-1,2,4-triazol-3-thion,
5-[(1,8-diethyl-1,3,4,9-tetrahydropyrano[3,4-*b*]indol-1-yl)methyl]-4-ethyl-2,4-dihydro-3H-1,2,4-triazol-3-thion,
5-[(1,8-diethyl-1,3,4,9-tetrahydropyrano[3,4-*b*]indol-1-yl)methyl]-4-propyl-2,4-dihydro-3H-1,2,4-triazol-3-thion,
5-[(1,8-diethy 1-1,3,4,9-tetrahydropyrano[3,4-*b*]indol-1-yl)methyl]-4-butyl-2,4-dihydro-3H-1,2,4-triazol-3-thion,
5-[(1,8-diethyl-1,3,4,9-tetrahydropyrano[3,4-*b*]indol-1-yl)methyl]-4-benzyl-2,4-dihydro-3H-1,2,4-triazol-3-thion,
5-[(1,8-diethyl-1,3,4,9-tetrahydropyrano[3,4-*b*]indol-1-yl)methyl]-4-allyl-2,4-dihydro-3H-1,2,4-triazole-3-thion,
5-[(1,8-diethy 1-1,3,4,9-tetrahydropyrano[3,4-*b*]indol-1-yl)methyl]-4-phenyl-2,4-dihydro-3H-1,2,4-triazol-3-thion, und
5-[(1,8-diethyl-1,3,4,9-tetrahydropyrano[3,4-*b*]indol-1-yl)methyl]-4-cyclohexyl-2,4-dihydro-3H-1,2,4-triazol-3-thion.

4. Eine Synthese Methode zur Herstellung von 5-[(1,8-diethyl-1,3,4,9-tetrahydropyrano[3,4-*b*]indol-1-yl)methyl]-4-substituiert-2,4-dihydro-3H-1,2,4-triazol-3-thion Zusammensetzungen gemäß Anspruch 3, **dadurch gekennzeichnet, dass** genannte Zusammensetzungen durch die Reaktion von 1-[2-(1,8-diethyl-1,3,4,9-tetrahydropyrano[3,4-*b*]indol-1-yl)acetyl]-4-alkyl/aryl thiosemicarbazid Zusammensetzungen synthetisiert werden, bei denen die alkyl/arly Gruppe aus einer aus der Gruppe von Methyl, Ethyl, Polyp, Butyl, Benzyl, Allyl, Phenyl und Cyclohexyl ausgewählt ist; in einem alkalischen Medium, gefolgt von Neutralisierung mit einer Säure.

## Revendications

1. Composé choisi dans le groupe constitué par :
le 1-[2-(1,8-diéthyl-1,3,4,9-tétrahydropyrano[3,4-b]indol-1-yl)acétyl]-4-méthyl-thiosemicarbazide,
le 1-[2-(1,8-diéthyl-1,3,4,9-tétrahydropyrano[3,4-b]indol-1-yl)acétyl]-4-éthyl-thiosemicarbazide,
le 1-[2-(1,8-diéthyl-1,3,4,9-tétrahydropyrano[3,4-b]indol-1-yl)acétyl]-4-propyl-thiosemicarbazide,
le 1-[2-(1,8-diéthyl-1,3,4,9-tétrahydropyrano[3,4-b]indol-1-yl)acétyl]-4-butyl-thiosemicarbazide,
le 1-[2-(1,8-diéthyl-1,3,4,9-tétrahydropyrano[3,4-b]indol-1-yl)acétyl]-4-benzyl-thiosemicarbazide,
le 1-[2-(1,8-diéthyl-1,3,4,9-tétrahydropyrano[3,4-b]indol-1-yl)acétyl]-4-allyl-thiosemicarbazide,
le 1-[2-(1,8-diéthyl-1,3,4,9-tétrahydropyrano[3,4-b]indol-1-yl)acétyl]-4-phényl-thiosemicarbazide, et
le 1-[2-(1,8-diéthyl-1,3,4,9-tétrahydropyrano[3,4-b]indol-1-yl)acétyl]-4-cyclohexyl-thiosemicarbazide.

2. Procédé de synthèse pour la production de composés de 1-[2-(1,8-diéthyl-1,3,4,9-tétrahydropyrano[3,4-b]indol-1-yl)acétyl]-4-alkyl/aryl-thiosemicarbazide selon la revendication 1, **caractérisé en ce que** du 2-(1,8-diéthyl-1,3,4,9-tétrahydropyrano[3,4-b]indol-1-yl)-acétohydrazide est mis à réagir avec des isothiocyanates substitués dans un milieu alcoolique.

3. Composé choisi dans le groupe constitué par :
la 5-[(1,8-diéthyl-1,3,4,9-tétrahydropyrano[3,4-b]indol-1-yl)méthyl]-4-méthyl-2,4-dihydro-3H-1,2,4-triazole-3-thione,
la 5-[(1,8-diéthyl-1,3,4,9-tétrahydropyrano[3,4-b]indol-1-yl)méthyl]-4-éthyl-2,4-dihydro-3H-1,2,4-triazole-3-thione,
la 5-[(1,8-diéthyl-1,3,4,9-tétrahydropyrano[3,4-b]indol-1-yl)méthyl]-4-propyl-2,4-dihydro-3H-1,2,4-triazole-3-thione,
la 5-[(1,8-diéthyl-1,3,4,9-tétrahydropyrano[3,4-b]indol-1-yl)méthyl]-4-butyl-2,4-dihydro-3H-1,2,4-triazole-3-thione,
la 5-[(1,8-diéthyl-1,3,4,9-tétrahydropyrano[3,4-b]indol-1-yl)méthyl]-4-benzyl-2,4-dihydro-3H-1,2,4-triazole-3-thione,
la 5-[(1,8-diéthyl-1,3,4,9-tétrahydropyrano[3,4-b]indol-1-yl)méthyl]-4-allyl-2,4-dihydro-3H-1,2,4-triazole-3-thione,
la 5-[(1,8-diéthyl-1,3,4,9-tétrahydropyrano[3,4-b]indol-1-yl)méthyl]-4-phenyl-2,4-dihydro-3H-1,2,4-triazole-3-thione, et
la 5-[(1,8-diéthyl-1,3,4,9-tétrahydropyrano[3,4-b]indol-1-yl)méthyl]-4-cyclohexyl-2,4-dihydro-3H-1,2,4-triazole-3-thione.

4. Procédé de synthèse pour la production de composés de 5-[(1,8-diéthyl-1,3,4,9-tétrahydropyrano[3,4-b]indol-1-yl)méthyl]-4-substitué-2,4-dihydro-3H-1,2,4-triazole-3-thione selon la revendication 3, **caractérisé en ce que** lesdits composés sont synthétisés par la réaction de composés de 1-[2-(1,8-diéthyl-1,3,4,9-tétrahydropyrano[3,4-b]indol-1-yl)acétyl]-4-alkyl/aryl-thiosemicarbazide où le groupe alkyle/aryle est choisi parmi les groupes méthyle, éthyle, propyle, butyle, benzyle, allyle, phényle et cyclohexyle; dans un milieu alcalin, puis par une neutralisation avec un acide.
